# EUROPÄISCHE PATENTSCHRIFT

(11) **EP 3 722 401 B1**
(45) Veröffentlichungstag und Bekanntmachung des Hinweises auf die Patenterteilung: **17.01.2024**
(21) Anmeldenummer: 19168321.8
(22) Anmeldetag: 10.04.2019
(51) Int. Cl.: C11D 1/72, C11D 1/83, C11D 17/00, E03D 9/03, E03D 9/02

(54) **GELFÖRMIGE SANITÄRMITTEL ZUR BIOFILM VERMEIDUNG**
GEL SANITARY AGENT FOR BIOFILM INHIBITION
MOYEN SANITAIRE SOUS LA FORME DE GEL PERMETTANT D'ÉVITER UN BIOFILM

(43) Veröffentlichungstag der Anmeldung: 14.10.2020
(73) Patentinhaber: Henkel AG & Co. KGaA, 40589 Düsseldorf (DE)
(72) Erfinder: Schymitzek, Tatiana, 47799 Krefeld (DE); Poethkow, Daniela, 47809 Krefeld (DE)

(56) Entgegenhaltungen:
- EP-A1- 1 029 911
- EP-A1- 1 894 989
- WO-A1-2014/033259
- DE-A1- 19 715 872
- DE-A1-102004 056 554

## Beschreibung

Aus dem Stand der Technik sind sanitäre Mittel in Gelform bekannt. Solche werden beispielsweise als selbsthaftende sanitäre Mittel verwendet. Diese selbsthaftenden sanitären Mittel sind geeignet, eigenständig, d.h. ohne weitere Hilfsmittel, an Oberflächen zu haften. Insbesondere sind aus dem Stand der Technik Toilettensteine, die eine selbsthaftende Zusammensetzung aufweisen, bekannt. Solche Toilettensteine können ohne Körbchen an die Wand der Toilettenschüssel angebracht werden und haften dort selbst nach einer Mehrzahl von Abspülvorgängen selbstständig. Beispielsweise ist in der Druckschrift EP 1086199B1 ein selbsthaftendes sanitäres Mittel in Form eines Gels beschrieben, welches selbstständig an Oberflächen von Gegenständen, die üblicherweise im sanitären Bereich verwendet werden, wie Toilettenschüsseln, haftet und erst nach mehreren Spülgängen von der Toilettenoberfläche abgespült wird.

In Toiletten kann es schnell zur Bildung von Biofilmen in der Spülschüssel kommen. Aus Hygienegründen ist es erwünscht, eine Biofilmbildung zu verhindern bzw. bereits bestehende Biofilme zu entfernen. Es wurde beobachtet, dass aus dem Stand der Technik bekannte gelförmige Sanitärmittel nur unzureichend geeignet sind, Biofilmbildung zu verhindern.

Insbesondere stellt die Vermeidung von Biofilmbildung unter gleichzeitiger Bereitstellung eines guten Schäumungsverhaltens eine Herausforderung dar.

Es war demnach Aufgabe der vorliegenden Erfindung, ein sanitäres Mittel bereitzustellen, das die Vermeidung von Biofilmbildung ermöglicht, bevorzugt ohne dass dabei die Schaumbildung beeinträchtigt wird.

Auch die DE 10 2004 056554 A1, die EP 1 894 989 A1 und die WO 2014/033259 A1 beschreiben solche Gele, die ohne Körbchen mittels eines Applikators an die Wand der Toilettenschüssel angebracht werden.

Ferner sind nicht-selbsthaftende Gele im Stand der Technik beschrieben. Diese Gele haften nicht an der Innenwand des Körbchens oder einer Toilettenschüssel und sind in DE 197 15 872 A1 und EP 1 029 911 A1 beschrieben. Diese Gele sind unter Krafteinwirkung verformbar.

Gemäß einem Aspekt wird ein System beschrieben, aufweisend:
ein WC Körbchen mit wenigstens einer Kammer, wobei ein selbsthaftendes gelförmiges sanitäreres Mittel in der Kammer des WC Körbchens vorliegt und an der Kammerhülle anhaftet und wobei die Kammerhülle auf wenigstens einer Seite vollständig geschlossen ist und das Mittel an der vollständig geschlossenen Seite der Kammerhülle anhaftet und wobei das sanitäre Mittel wenigstens einen nicht-ionischen Haftvermittler aufweist und wobei der nicht-ionische Haftvermittler wenigstens einen hydrophoben Rest aufweist und ferner eine hydrophile Gruppe umfasst, wobei das sanitäre Mittel sauer ist.

Gemäß einer bevorzugten Ausführungsform wird ein sanitäres Mittel bereitgestellt, wobei das sanitäre Mittel ferner ein anionisches Tensid aufweist.

Gemäß einer bevorzugten Ausführungsform wird ein sanitäres Mittel bereitgestellt, wobei das anionische Tensid ein Ethersulfat, bevorzugt ein Fettsäureethylethersulfat und besonders bevorzugt ein Natriumlaurylethersulfat ist.

Gemäß einer bevorzugten Ausführungsform wird ein sanitäres Mittel bereitgestellt, wobei das anionische Tensid in einer Menge von 1 Gew.-% bis 20 Gew.-%, bevorzugt in einer Menge von 2 Gew.-% bis 10 Gew.-%, bezogen auf die Gesamtmenge des Mittels vorliegt.

Gemäß einer bevorzugten Ausführungsform wird ein Mittel bereitgestellt, wobei der nicht-ionische Haftvermittler ein Fettalkoholalkoxylat ist, das bevorzugt einen Alkoxylierungsgrad von 20 bis 50 EO, bevorzugt von 20 bis 35 aufweist.

Gemäß einer bevorzugten Ausführungsform wird ein sanitäres Mittel bereitgestellt, wobei das Mittel ein weiteres nicht-ionisches Tensid aufweist. Gemäß einer bevorzugten Ausführungsform wird ein Mittel bereitgestellt, wobei das weitere nicht-ionische Tensid ein Fettalkoholalkoxylat ist, bevorzugt mit einem Alkoxylierungsgrad von 5 bis 15 EO. Gemäß einer bevorzugten Ausführungsform wird ein Mittel bereitgestellt, wobei das Fettalkoholalkoxylat (weiteres nicht-ionisches Tenisid) einen C9 bis C12 Alkylrest aufweist, und wobei der Alkyrest bevorzugt ein unverzweigter Alkylrest ist.

Im Zusammenhang mit der vorliegenden Erfindung wird der Ausdruck "sanitäres Mittel" im Sinne einer "Zusammensetzung, die geeignet ist zur Verwendung als sanitäres Mittel", verwendet. Sofern im Zusammenhang mit der vorliegenden Erfindung von "sanitärem Mittel" oder einfach nur "Mittel" gesprochen wird, ist, sofern nichts anderes zu entnehmen ist, eine Gelzusammensetzung gemeint. Im Zusammenhang mit der vorliegenden Erfindung steht der Ausdruck "EO" für Alkoxygruppen im Allgemeinen. So bedeutet beispielsweise der Ausdruck "20 EO", die Anwesenheit von 20 Alkoxygruppen, die besonders bevorzugt als Block im betreffenden Molekül vorliegen und noch weiter bevorzugt als Endgruppen im betreffenden Molekül angeordnet sind. Insbesondere steht der Ausdruck "EO" für die Gruppe enthaltend Ethoxy-, Propoxy- und Butoxygruppen, weiter bevorzugt für die Gruppe enthaltend Ethoxy- und Propoxygruppen und besonders bevorzugt nur für Ethoxygruppen.

Im Zusammenhang mit der vorliegenden Erfindung wird unter "Sanitärgegenstand" bevorzugt ein Gegenstand verstanden, der üblicherweise im Bereich von privaten oder öffentlichen Bädern und Toiletten Verwendung findet. Insbesondere sind unter Sanitärgegenstand Toiletten, bevorzugt Toilettenschüsseln, Urinale, Pissoirs, aber auch Handspülbecken zu verstehen.

Im Zusammenhang mit der vorliegenden Erfindung werden die Ausdrücke "lipophil" bzw. "Lipophilie" synonym zu den Ausdrücken "hydrophob" bzw. "Hydrophilie" benutzt.

Im Zusammenhang mit der vorliegenden Erfindung wird unter "fließbar machen" verstanden, dass die Zusammensetzung erhitzt wird bis zu einem Punkt, an dem diese eine Viskosität erreicht hat, bei der die Zusammensetzung derart fließfähig ist, dass die Zusammensetzung ausgegossen werden kann und damit in ein anderes Behältnis, insbesondere die Kammer eines WC-Körbchens, umgefüllt werden kann.

Die Zusammensetzung kann Polyole, bevorzugt Glycerin, in einer Menge von 3 Gew.-% bis 35 Gew.-%, insbesondere 5 bis 15 Gew.-% enthalten.

Der Gelzusammensetzung ist ein Bestandteil des erfindungsgemäßen sanitären Mittels und weist einen nicht-ionischen Haftvermittler auf.

Der Haftvermittler bewirkt in Anwesenheit von Wasser, dass das Mittel an einer Oberfläche haftet und bevorzugt netzwerkartige Strukturen ausbildet. Diese verleihen dem Mittel selbst bei Spülung in einer Toilette die erforderliche Formbeständigkeit. Das sanitäre Mittel ist bevorzugt selbsthaftend ausgebildet, d.h. das sanitäre Mittel ist geeignet eigenständig, insbesondere ohne weitere Hilfsmittel, an Oberflächen zu haften.

Der Haftvermittler kann entweder insgesamt hydrophil sein, bevorzugt aber teilweise hydrophil und teilweise hydrophob, d.h. die Moleküle des Haftvermittlers sind bevorzugt längerkettige Moleküle, die wenigstens einen hydrophoben Rest aufweisen und ferner eine hydrophile Gruppe umfassen, die mit einem bevorzugt polaren Lösungsmittel, insbesondere mit Wasser, wechselwirkt. Vorzugsweise handelt es sich bei den Haftvermittlern um unverzweigte Moleküle, d.h. lineare Moleküle. Dies ermöglicht die gewünschte Netzwerkbildung.

Als hydrophile Reste können Polyalkoxygruppen, vorzugsweise Polyethoxy-, Polypropoxy- oder Polybutoxy- oder auch gemischte Polyalkoxygruppen wie beispielsweise Poly(ethoxypropoxy)gruppen eingesetzt werden. Ein Fettalkoholalkoxylat, welches sowohl Ethoxy also auch Propoxygruppen aufweist, wird im Zusammenhang mit der Erfindung als ein gemischtes Fettalkoholethoxypropoxylat bezeichnet.

Besonders bevorzugt als hydrophile Gruppe ist ein Polyethoxyrest, der weiter bevorzugt zwischen 15 und 55 Ethoxygruppen, weiter vorzugsweise zwischen 20 und 50 und besonders bevorzugt zwischen 22 und 28 Ethoxygruppen umfasst. Ferner haben sich auch Polyethoxyreste mit bevorzugt zwischen 35 und 50 Ethoxygruppen als zweckmäßig ergeben.

Als hydrophober Rest sind bevorzugt lineare, d.h. unverzweigte Alkylreste geeignet. Hierdurch wird die Eignung zur Netzwerkbildung begünstigt. Geradzahlige Alkylreste sind bevorzugt - wegen deren besseren biologischen Abbaubarkeit. Besonders bevorzugt sind Alkylreste mit wenigstens mehr als elf Kohlenstoffatomen. Weiter bevorzugt beträgt die Zahl der Kohlenstoffatome 9 bis 30 insbesondere 7 bis 25.

Bevorzugte Haftvermittler sind Polyalkoxyalkane, insbesondere ein Gemisch aus Alkyl-ethoxylat mit 20 bis 50 EO, insbesondere 25 EO oder aber 40 EO, wobei die Alkylkette weiter bevorzugt C16-25 Kohlenstoffatome aufweist.

Mit Verringerung der Anzahl der Alkoxygruppen wird der Haftvermittler lipophiler. Hierdurch lässt sich insbesondere die Löslichkeit von hydrophilen Substanzen, wie beispielsweise Duftstoffen oder Farbstoffen, regulieren.

Mit Erhöhung des Grades der Alkoxylierung wird der Haftvermittler hydrophiler, was insbesondere die Ausbildung eines Netzwerkes und die Fähigkeit zur Bildung eines optisch ansprechenden Gels beeinflussen kann und darüber hinaus einen Einfluss auf das Abspülverhalten des sanitären Mittels haben kann.

Ebenfalls Auswirkungen auf die Lipophilie des Haftvermittlers hat die Art und insbesondere die Länge des hydrophoben Restes, wobei insbesondere längere Alkylreste die Hydrophobie des Haftvermittlers erhöhen. Insgesamt lässt sich durch geeignete Wahl des hydrophoben Restes, insbesondere eines linearen, d. h. unverzweigten, Alkylrestes auf der einen Seite, in Abstimmung mit dem Grad der Alkoxylierung, d. h. der Anzahl an Alkoxygruppen, ein maßgeschneiderter Haftvermittler bereitstellen, der im Hinblick auf das Abspülverhalten des sanitären Mittels, die Haftfähigkeit an Oberflächen, sowie im Hinblick auf die Löslichkeit von hydrophoben oder hydrophilen Zusatzstoffen optimiert ist.

Auch die einzusetzende Menge an Haftvermittler ist von dessen Hydrophilie und dessen Netzwerkbildungsvermögen abhängig. Diese beträgt bevorzugt 10 und 40 Gew.-%, was insbesondere bei der Verwendung von Polyalkoxyalkanen bevorzugt ist.

"Fettalkoholalkoxylate" im Sinne der vorliegenden Erfindung sind Verbindungen, die wenigstens einen hydrophoben Rest und wenigstens einen hydrophilen Rest umfassen, wobei der hydrophile Rest wenigstens eine EO Einheit ist, die an den hydrophoben Rest gebunden ist. Der hydrophobe Rest leitet sich insbesondere von aliphatischen, langkettigen, einwertigen Alkoholen ab. Diese können natürlich oder synthetisch vorkommen, wobei natürliche Alkohole bevorzugt sind. Der hydrophobe Rest ist bevorzugt verzweigt. Bevorzugt weist der hydrophobe Rest zwischen 6 bis 22 Kohlenstoffatome auf und kann auch ein- oder mehrfach ungesättigt sein, wobei vollständig gesättigte Reste bevorzugt sind.

Das Fettalkoholalkoxylat weist bevorzugt einen Alkoxylierungsgrad von 10 bis 50 EO, insbesondere von 20 bis 45 EO, auf. Alternativ weist das Fettalkoholalkoxylat einen Alkoxylierungsgrad von 5 bis 15 EO auf.

Weiter bevorzugt umfasst die Gelzusammensetzung ein erstes Fettalkoholalkoxylat mit einem Alkoxylierungsgrad von 10 bis 50 EO, insbesondere von 20 bis 45, EO und ein zweites Fettalkoholalkoxylat mit einem Alkoxylierungsgrad von 5 bis 15 EO.

Insbesondere kann die Gelzusammensetzung ein erstes Fettalkoholalkoxylat mit einem Alkoxylierungsgrad von 20 bis 30 EO in einer Menge von 15 bis 40 Gew.-% (als Haftvermittler) und ein zweites Fettalkoholalkoxylat mit einem Alkoxylierungsgrad von 5 bis 15 EO in einer Menge von 2 bis 20 Gew.-% (als weiteres nicht-ionisches Tensid) umfassen.

Bevorzugt ist das Fettalkoholalkoxylat ein Fettalkoholethoxylat, wobei das Fettalkoholalkoxylat wenigstens einen Ethoxyrest aufweist und/oder ein Fettalkoholpropoxylat, wobei das Fettalkoholalkoxylat wenigstens einen Propoxyrest aufweist. Weiter bevorzugt umfasst das Fettalkoholalkoxylat ausschließlich Ethoxy und/oder Propoxygruppen als Alkoxygruppen. Noch weiter bevorzugt umfasst das Fettalkoholalkoxylat ausschließlich Ethoxygruppen als Alkoxygruppen. Bevorzugt weist dabei der Alkyrest wenigstens 7 Kohlenstoffatome auf, insbesondere 7 bis 20 Kohlenstoffatome, wobei der Alkyrest insbesondere ein unverzweigter Alkylrest ist.

Gemäß einer bevorzugten Ausführungsform wird ein sanitäres Mittel beschrieben, wobei das Mittel einen sauren pH - Wert, gemessen in einer 10-% Lösung des Mittels in Wasser, aufweist, und wobei das Mittel ferner ein erstes Fettalkoholalkoxylat mit einem Alkoxylierungsgrad von 20 bis 30 EO in einer Menge von 15 bis 40 Gew.-% (als Haftvermittler) und ein zweites Fettalkoholalkoxylat mit einem Alkoxylierungsgrad von 5 bis 15 EO in einer Menge von 2 bis 20 Gew.-% (als weiteres nicht-ionisches Tensid) umfasst.

Gemäß einer weiter bevorzugten Ausführungsform wird ein sanitäres Mittel beschrieben, wobei das Mittel einen sauren pH - Wert, gemessen in einer 10-% Lösung des Mittels in Wasser, aufweist, und wobei das Mittel ferner ein erstes Fettalkoholalkoxylat mit einem Alkoxylierungsgrad von 20 bis 30 EO in einer Menge von 15 bis 40 Gew.-% (als Haftvermittler) und ein zweites Fettalkoholalkoxylat mit einem Alkoxylierungsgrad von 5 bis 15 EO in einer Menge von 2 bis 20 Gew.-% (als weiteres nicht-ionisches Tensid) sowie ein anionisches Tensid in einer Menge von 2 bis 10 Gew.-% umfasst.

Gemäß einer besonders bevorzugten Ausführungsform wird ein sanitäres Mittel beschrieben, aufweisend wenigstens einen nicht-ionischen Haftvermittler, wobei das Mittel einen pH - Wert von 2 bis 6, bevorzugt von 3 bis 5, aufweist, gemessen in einer 10-% Lösung des Mittels in Wasser, und wobei das Mittel ein anionisches Tensid aufweist, welches bevorzugt ein Ethersulfat, ist, bevorzugt ein Fettsäureethylethersulfat und besonders bevorzugt ein Natriumlaurylethersulfat, und wobei das anionische Tensid in einer Menge von 1 Gew.-% bis 20 Gew.-%, bevorzugt in einer Menge von 2 Gew.-% bis 10 Gew.-%, bezogen auf die Gesamtmenge des Mittels vorliegt, und wobei der nicht-ionische Haftvermittler ein Fettalkoholalkoxylat ist, das bevorzugt einen Alkoxylierungsgrad von 20 bis 50 EO, bevorzugt von 20 bis 35 aufweist und in einem Menge von 15 bis 35 Gew.-% vorliegt und das Mittel ferner ein weiteres nicht-ionisches Tensid aufweist, wobei das weitere nicht-ionische Tensid ein Fettalkoholalkoxylat ist, mit einem Alkoxylierungsgrad von 5 bis 15 EO und einen C9 bis C12 Alkylrest aufweist und das weitere nicht-ionische Tensid in einer Menge von 2 bis 12 Gew.-% in der Zusammensetzung vorliegt.

Gemäß einer weiteren besonders bevorzugten Ausführungsform wird ein sanitäres Mittel beschrieben, aufweisend wenigstens einen nicht-ionischen Haftvermittler, wobei das Mittel einen pH - Wert von 2 bis 6, bevorzugt von 3 bis 5, aufweist, gemessen in einer 10-% Lösung des Mittels in Wasser und wobei das Mittel ein Natriumlaurylethersulfat in einer Menge von 3 bis 4 Gew.-% aufweist und wobei der nicht-ionische Haftvermittler ein Fettalkoholalkoxylat mit 20 bis 30 EO (Ethoxy) ist und in einem Menge von 25 bis 35 Gew.-% vorliegt und das Mittel ferner ein weiteres nicht-ionisches Tensid aufweist, wobei das weitere nicht-ionische Tensid ein Fettalkoholalkoxylat ist, mit einem Alkoxylierungsgrad von 5 bis 10 EO (Ethoxy), und einen C9 bis C12 Alkylrest aufweist und in einer Menge von 8 bis 12 Gew.-% vorliegt.

Alternativ zu dieser Ausführungsform und ebenfalls bevorzugt wird ein sanitäres Mittel beschrieben, aufweisend wenigstens einen nicht-ionischen Haftvermittler, wobei das Mittel einen pH - Wert von 2 bis 6, bevorzugt von 3 bis 5, aufweist, gemessen in einer 10-% Lösung des Mittels in Wasser und wobei das Mittel ein Natriumlaurylethersulfat in einer Menge von 5 bis 10 Gew.-% aufweist und wobei der nicht-ionische Haftvermittler ein Fettalkoholalkoxylat mit 20 bis 30 EO (Ethoxy) ist und in einem Menge von 15 bis 25 Gew.-% vorliegt und das Mittel ferner ein weiteres nicht-ionisches Tensid aufweist, wobei das weitere nicht-ionische Tensid ein Fettalkoholalkoxylat ist, mit einem Alkoxylierungsgrad von 5 bis 10 EO (Ethoxy), und einen C9 bis C12 Alkylrest aufweist und in einer Menge von 2 bis 7 Gew.-% vorliegt.

Die Art und Menge des Lösungsmittels ist unter anderem von der Hydrophilie des eingesetzten Haftvermittlers abhängig und kann entsprechend variiert werden. Um mit den Haftvermittlermolekülen die gewünschte Anzahl von Klebestellen bereitzustellen, sollte das Lösungsmittel wenigstens zu 20 Gew.-%, vorzugsweise zwischen 35 und 75 Gew.-% in Bezug auf die Gelzusammensetzung enthalten sein.

Bevorzugt ist das Lösungsmittel ein polares Lösungsmittel, weiter bevorzugt ein wässriges Lösungsmittel, und insbesondere Wasser. Insbesondere umfasst die Gelzusammensetzung Wasser in einer Menge zwischen 40 und 75 Gew.-% bezogen auf die Gesamtmenge der Gelzusammensetzung.

Die Gelzusammensetzung umfasst bevorzugt Duft- und/oder Riechstoffe. Hierdurch kann die Raumluft verbessert werden.

Die Gelzusammensetzung umfasst bevorzugt Konservierungsstoffe. Solche Konservierungsstoffe sind beispielsweise unter den Handelsnamen Acticide B 20, Acticide MBR 1 und Acticide SR 1500 erhältlich. Bevorzugt umfasst die Gelzusammensetzung als Konservierungsstoffe eines oder mehrere aus der Gruppe enthaltend Isothiazolinon, Phenoxyethanol, Methylisothiazolinon und Benzisothiazolinone (BIT). Bevorzugt und abhängig von der Art des Konservierungsstoffes ist dieser in Mengen von 0,0001 bis 1 wt.-% in der Gelzusammensetzung enthalten.

Vorzugsweise weist das erfindungsgemäße Mittel eine salbenartige, pastöse und/oder eine cremeartige Konsistenz auf.

Im Zusammenhang mit der vorliegenden Erfindung wird unter "Gel" bevorzugt ein disperses System verstanden, das aus mindestens zwei Komponenten besteht. Die feste Komponente bildet dabei ein schwammartiges, dreidimensionales Netzwerk, dessen Poren zumindest teilweise durch ein Lösungsmittel ausgefüllt sind.

Bevorzugt sind die sanitären Mittel als Gele ausgebildet. Solche Gele weisen bevorzugt eine andere Mikro- beziehungsweise Nanostruktur auf als herkömmliche sanitäre Mittel, was insbesondere im Falle von Toilettenanwendungen nicht nur zu einer besonderen optischen Erscheinung aufgrund der Transparenz des Gels führt, sondern auch eine gleichmäßige Freisetzung von Additiven wie Farbstoffen und insbesondere Duftstoffzusammensetzungen ermöglicht.

Ein weiterer Aspekt der Erfindung betrifft die Verwendung des sanitären Mittels, wobei das Mittel auf einem Sanitärgegenstand angehaftet wird. Bevorzugt umfasst die Verwendung die Schritte: (a) Bereitstellen des sanitären Mittels; und (b) Anhaften des sanitären Mittels an einer Oberfläche eines Sanitärgegenstandes.

Das Anhaften des sanitären Mittels gemäß dieser Verwendung findet bevorzugt derart statt, dass dieses unmittelbar auf der Oberfläche des Sanitärgegenstandes haftet. Anders ausgedrückt haftet das sanitäre Mittel selbstständig auf der Oberfläche des Sanitärgegenstandes.

In einer bevorzugten Ausführungsform wird das Anhaften des sanitären Mittels mittels eines Applikators durchgeführt. Hierzu eignen sich beispielsweise Spritzen im weitesten Sinne, die das sanitäre Mittel enthalten und aus denen das sanitäre Mittel herausgedrückt werden kann, um dieses auf der Oberfläche des Sanitärgegenstandes zu platzieren.

Die erreichte Haftung an dem Sanitärgegenstand, bevorzugt selbst bei einer Anbringung an einer senkrechten Fläche, ist derart, dass sich das Mittel sogar unter der zusätzlichen Krafteinwirkung von Spülwasserströmen nicht auf einmal ablöst.

Das sanitäre Mittel ist bevorzugt erst nach einer größeren Anzahl von Spülvorgängen abspülbar. Die Anzahl der Spülvorgänge richtet sich nach der Zusammensetzung des jeweiligen sanitären Mittels und ferner nach der aufgebrachten Menge sowie der Form des sanitären Mittels.

Das sanitäre Mittel kann auch gleichzeitig an mehreren Stellen des Sanitärgegenstands angehaftet werden. Beispielsweise kann das Mittel sowohl auf der rechten als auch auf der linken Seite einer Toilettenschüssel aufgebracht werden. Dies ermöglicht eine gleichmäßigere Reinigungswirkung als die Anbringung nur auf einer Seite.

Gemäß einer weiteren Ausführungsform kann das sanitäre Mittel an einem Sanitärgegenstand an verschiedenen Stellen in unterschiedlichen Zusammensetzungen angehaftet werden. Dies ermöglicht beispielsweise, dass z.B. zwei unterschiedliche Parfümierungsstoffe bei örtlicher Trennung zu einer gemeinsamen Beduftung der Toilette eingesetzt werden können.

Darüber hinaus haben die Erfinder festgestellt, dass das hier beschriebene sanitäre Mittel auch vorteilhaft in einem WC Körbchen vorgesehen sein kann. Ein weiterer Aspekt betrifft ein System aufweisend das sanitäre Mittel gemäß der Erfindung und ein WC Körbchen mit wenigstens einer Kammer, wobei das sanitäre Mittel in der Kammer des WC Körbchens vorliegt.

Ein weiterer Aspekt der Erfindung betrifft die Herstellung des erfindungsgemäßen Systems umfassend die Schritte (a) Fließbarmachen der Gelzusammensetzung durch Erhitzen, (b) Einbringen des Mittels in das Körbchen, (c) Abkühlen der Zusammensetzung, wobei das Mittel aushärtet.

In einer bevorzugten Ausführungsform ist die Kammerhülle des WC Körbchens auf wenigstens einer Seite vollständig geschlossen und die Zusammensetzung an der vollständig geschlossenen Seite der Kammerhülle angehaftet.

Dabei ist das sanitäre Mittel bevorzugt als Gel ausgebildet. Eine solches Mittel in einem WC Körbchen in Form eines Gels ist ein optisch attraktives, transparentes Produkt mit einer vorteilhaften Freisetzungscharakteristik in Bezug auf Farb- und insbesondere Duftstoffe die in der Zusammensetzung als Additive enthalten sein können.

Durch die selbsthaftenden Eigenschaften des Gels haftet dieses an der Oberfläche des WC Körbchens. Dies hat den Vorteil, dass das im Körbchen enthaltene Gel nicht wie bei üblichen Toilettensteinen von außen nach innen aufgelöst wird, sondern ein Auflöseverhalten dahingehend aufweist, dass die Mengen an Gel, die unmittelbar an der Kammerwand des WC Körbchens haften, zuletzt abgespült werden. Hierdurch ergibt sich insbesondere ein verbessertes Freisetzungsprofil sowie ein optisch ansprechenderes Erscheinungsbild über die gesamte Lebensdauer des WC-Steines. Da sich die von außen für den Benutzer erkennbaren Bereiche des Gels, zum Beispiel bei Verwendung eines transparenten WC Körbchens, erst ganz am Ende der Lebenszeit ablösen, wird sich das ursprüngliche Erscheinungsbild des WC Steines bis zum vollständigen Aufbrauchen des Gleichen kaum ändern. Dies stellt allerdings besondere Anforderungen an das Abspülverhalten des Gels von der Oberfläche der Kammer, in welcher das Gel im WC Körbchen enthalten ist.

Ein unzureichendes Abspülverhalten von der inneren Kammeroberfläche des WC Körbchens führt bei Verwendung von transparenten WC Körbchen zu unansehnlichen da ungleichmäßig verteilten Gelresten an der Oberfläche der Kammer. WC Körbchen weisen in der Regel eine stark gebogene bzw. gekrümmte innere Kammeroberfläche auf (beispielsweise bei sphärisch ausgebildeten WC Körbchen). Gegenüber den relativ glatten und ungekrümmten Oberflächen von Toilettenschüsseln, an denen sanitäre Mittel direkt ohne WC Körbchen angebracht werden können, ist bei Verwendung von WC Körbchen mit einem selbsthaftenden Gel eine ausgeprägtere Rückstandsbildung zu beobachten. Eine Verbesserung des Abspülverhalten ist somit insbesondere bei Verwendung mit einem WC-Körbchen notwendig.

Bevorzugt ist die Kammerhülle als Kugel, d.h. sphärisch ausgebildet, wobei die Kammerhülle einstückig oder zweistückig aus einer ersten Halbkugel und einer zweiten Halbkugel besteht, die respektive eine erste Seite der Kammerhülle und eine zweite Seite der Kammerhülle darstellen. Die erste Seite kann als Vorderseite bezeichnet werden, d.h. die im Benutzungszustand zugängliche bzw. sichtbare und der Toilettenschüsselwand abgewandte Seite, während die zweite Seite als Rückseite bezeichnet werden kann, d.h. die im Benutzungszustand nicht unmittelbar zugängliche bzw. sichtbare und der Toilettenschüsselwand und dem daran entlanglaufenden Spülwasser zugewandte Seite. Die vorstehende Einteilung der Kammerhülle in zwei Seiten kann auch auf weitere Kammerhüllengeometrien, z.B. rechteckige Kammerhüllengeometrien, entsprechend übertragen werden.

Die Vorderseite der Kammerhülle weist bevorzugt keine Öffnung auf. Dies verhindert zum einen, dass ein Kind oder ein Haustier in Kontakt mit einer in der Kammer vorhandenen Wirksubstanz geraten kann. Ferner kann dadurch, dass die erste Seite der Kammerhülle keine Öffnung aufweist, die Wirksubstanz beim Herstellungsprozess durch eine Öffnung der zweiten Seite in das Körbchen eingefüllt werden. Bei WC Körben mit einer oder mehrerer Öffnungen auf der ersten Seite würde die Wirksubstanz bei der Befüllung nicht im Körbchen verbleiben, sondern durch dieses hindurchlaufen. Vorteilhaft kann ein WC Körbchen gemäß der beschriebenen Ausführungsform mit einer Wirksubstanz befüllt werden, wobei die Substanz sich erst nach der Befüllung verfestigt. Es ist also nicht notwendig, dass die Wirksubstanz zu der gewünschten Geometrie (z.B. einer Kugel) vorgeformt wird und erst dann in die Kammer eingebracht wird. Vielmehr wird die Formgebung dadurch erreicht, dass die Wirksubstanz in die Kammer eingefüllt wird und die Kammergeometrie bei der Verfestigung der Wirksubstanz als Matrize fungiert. Insbesondere ermöglicht die beschriebene Ausführungsform, pastöse Mittel, als Wirksubstanzen einzusetzen, da solche pastösen gelförmigen Mittel, wegen Ihrer Konsistenz und "Klebrigkeit" nur schwer vorgeformt und in ihrer endgültigen Form in die Kammer des Körbchens eingebracht werden können.

Im Falle der erfindungsgemäßen Mittel konnte beobachtet werden, dass diese schnell ohne Anwendung zusätzlicher Kühlung erstarren bzw. aushärten. Der hierdurch bedingte verminderte Energieaufwand verursacht weniger Kosten und ermöglicht eine ökologischere und effizientere großindustrielle Herstellung der sanitären Mittel.

Ein weiterer Aspekt der Erfindung betrifft die Verwendung des Systems umfassend die Schritte: (a) Bereitstellen des Systems aufweisend das sanitäre Mittel gemäß der Erfindung und ein WC Körbchen mit wenigstens einer Kammer, wobei das sanitäre Mittel in der Kammer des WC Körbchens vorliegt (b) Anbringen des Systems an einem Sanitärgegenstand.

### Beispiele:

Es wurden die Mittel E1 bis E4 bereitgestellt. Zum Vergleich wurde darüber hinaus ein Mittel V1 bereitgestellt, welches nur nicht-ionische Tenside aufweist, allerdings kein anionisches Tensid.

Die Mengen sind in Gewichtsprozent Gew.-% bezogen auf die Gesamtmenge der Zusammensetzung angegeben:

| | **V1** | **E1** | **E2** | **E3** | **E4** |
|---|---|---|---|---|---|
| Ceteareth-25 EO | 30 | 30 | 20 | 30 | 20 |
| Fettalkoholethoxylat: C9 bis C12 Alkylrest; 8 EO | 15 | 10 | 5 | 10 | 5 |
| Natriumlaurethsulfat | 0 | 3,5 | 7 | 3,5 | 7 |
| Duftstoffe | <1 | <1 | <1 | <1 | <1 |
| Farbstoffe | <1 | <1 | <1 | <1 | <1 |
| Konservierungmittel | <1 | <1 | <1 | <1 | <1 |
| Wasser | Rest | Rest | Rest | Rest | Rest |
| pH Wert | neutral | neutral | neutral | sauer | sauer |
| Schäumungsverhalten | - | ++ | ++ | ++ | ++ |
| Vermeidung Biofilm | - | - | - | ++ | ++ |

Die Beispiele E1 und E2 sind nicht erfindungsgemäss. Für die beschriebenen Beispiele wurden die Gelzusammensetzungen in WC Körbchen eingebracht und am Rand der Toilettenschüssel befestigt.

In Toiletten kann es schnell zur Bildung von Biofilmen in der Spülschüssel kommen. Aus Hygienegründen ist es erwünscht, eine Biofilmbildung zu verhindern bzw. bereits bestehende Biofilme zu entfernen. Es wurde beobachtet, dass die neutralen Gelzusammensetzungen nur unzureichend geeignet sind, Biofilmbildung zu verhindern. Die sauren Zusammensetzungen hingegen verhindern die Biofilmbildungen. Sofern auch ein anionisches Tensid verwendet wird, kann gleichzeitig eine gutes Schäumungsverhalten und eine Vermeidung von Biofilmbildung erreicht werden.

## Patentansprüche

1. System aufweisend
ein WC Körbchen mit wenigstens einer Kammer, wobei ein selbsthaftendes gelförmiges sanitäreres Mittel in der Kammer des WC Körbchens vorliegt und an der Kammerhülle anhaftet und wobei
die Kammerhülle auf wenigstens einer Seite vollständig geschlossen ist und das Mittel an der vollständig geschlossenen Seite der Kammerhülle anhaftet und wobei das sanitäre Mittel wenigstens einen nicht-ionischen Haftvermittler aufweist und wobei der nicht-ionische Haftvermittler wenigstens einen hydrophoben Rest aufweist und ferner eine hydrophile Gruppe umfasst, wobei das sanitäre Mittel sauer ist.

2. System gemäß Anspruch 1, wobei das Mittel einen pH - Wert von 2 bis 6, bevorzugt von 3 bis 5, aufweist, gemessen in einer 10-% Lösung des Mittels in Wasser.

3. System gemäß einem der beiden vorhergehenden Ansprüche, wobei das sanitäre Mittel ferner ein anionisches Tensid aufweist.

4. System gemäß dem vorhergehenden Anspruch, wobei das anionische Tensid ein Ethersulfat, bevorzugt ein Fettsäureethylethersulfat und besonders bevorzugt ein Laurylethersulfat ist.

5. System gemäß einem der beiden vorhergehenden Ansprüche, wobei das anionische Tensid in einer Menge von 1 Gew.-% bis 20 Gew.-%, bevorzugt in einer Menge von 2 Gew.-% bis 10 Gew.-%, bezogen auf die Gesamtmenge des Mittels vorliegt.

6. System gemäß einem der vorhergehenden Ansprüche, wobei der nicht-ionische Haftvermittler ein Fettalkoholalkoxylat ist, das bevorzugt einen Alkoxylierungsgrad von 20 bis 50 EO, bevorzugt von 20 bis 45 aufweist.

7. System gemäß einem der vorhergehenden Ansprüche, wobei Mittel ein weiteres nicht-ionisches Tensid aufweist.

8. System gemäß dem vorhergehenden Anspruch, wobei das weitere nicht-ionische Tensid ein Fettalkoholalkoxylat ist, bevorzugt mit einem Alkoxylierungsgrad von 5 bis 15 EO.

9. System gemäß dem vorhergehenden Anspruch, wobei das Fettalkoholalkoxylat einen C9 bis C12 Alkylrest aufweist, und wobei der Alkyrest bevorzugt ein unverzweigter Alkylrest ist.

10. Herstellung eines Systems gemäß dem vorhergehenden Anspruch, umfassend die Schritte, Fließbarmachen des Gels durch Erhitzen,
Einbringen des Mittels in das Körbchen,
Abkühlen der Zusammensetzung, wobei das Mittel aushärtet.

## Claims

1. A system comprising
a toilet bowl having at least one chamber, wherein a self-adhesive gel-like sanitizing agent is present in the chamber of the toilet bowl and adheres to the chamber shell, and wherein the chamber shell is completely closed on at least one side and the agent adheres to the completely closed side of the chamber shell and wherein the sanitizing agent comprises at least one non-ionic adhesion promoter and wherein the non-ionic adhesion promoter comprises at least one hydrophobic moiety and further comprises a hydrophilic group, wherein the sanitizing agent is acidic.

2. System according to claim 1, wherein the agent has a pH value of from 2 to 6, preferably from 3 to 5, measured in a 10% solution of the agent in water.

3. System according to any one of the two preceding claims, wherein the sanitizing agent further comprises an anionic surfactant.

4. System according to the preceding claim, wherein the anionic surfactant is an ether sulfate, preferably a fatty acid ethyl ether sulfate and particularly preferably a lauryl ether sulfate.

5. System according to one of the two preceding claims, wherein the anionic surfactant is present in an amount of from 1% to 20% by weight, preferably in an amount of from 2% to 10% by weight, based on the total amount of the composition.

6. system according to one of the preceding claims, wherein the non-ionic adhesion promoter is a fatty alcohol alkoxylate, preferably having a degree of alkoxylation of from 20 to 50 EO, preferably from 20 to 45.

7. System according to any one of the preceding claims, wherein the agent comprises a further non-ionic surfactant.

8. System according to the preceding claim, wherein the further non-ionic surfactant is a fatty alcohol alkoxylate, preferably with a degree of alkoxylation of 5 to 15 EO.

9. System according to the preceding claim, wherein the fatty alcohol alkoxylate comprises a C9 to C12 alkyl radical, and wherein the alkyl radical is preferably an unbranched alkyl radical.

10. Manufacturing a system according to the preceding claim, comprising the steps of,
Make the gel flowable by heating,
Insert the product into the basket,
Cooling of the composition, whereby the agent hardens.

## Revendications

1. Système comprenant
un panier de WC avec au moins un compartiment, dans lequel un agent sanitaire en forme de gel auto-adhésif est présent dans le compartiment du panier de WC et adhère à l'enveloppe du compartiment, et dans lequel
l'enveloppe de chambre est complètement fermée sur au moins un côté et l'agent adhère au côté complètement fermé de l'enveloppe de chambre et dans lequel l'agent sanitaire comprend au moins un agent adhésif non ionique et dans lequel l'agent adhésif non ionique comprend au moins un résidu hydrophobe et comprend en outre un groupe hydrophile, dans lequel l'agent sanitaire est acide.

2. Système selon la revendication 1, dans lequel l'agent a un pH de 2 à 6, de préférence de 3 à 5, mesuré dans une solution à 10 % de l'agent dans l'eau.

3. Système selon l'une des deux revendications précédentes, dans lequel l'agent sanitaire comprend en outre un agent tensioactif anionique.

4. Système selon la revendication précédente, dans lequel le tensioactif anionique est un éther sulfate, de préférence un éthyléther sulfate d'acide gras et de manière particulièrement préférée un lauryl éther sulfate.

5. Système selon l'une des deux revendications précédentes, dans lequel le tensioactif anionique est présent en une quantité allant de 1 % à 20 % en poids, de préférence en une quantité allant de 2 % à 10 % en poids, par rapport à la quantité totale de la composition.

6. Système selon l'une des revendications précédentes, dans lequel l'agent de couplage non ionique est un alcoxylate d'alcool gras, présentant de préférence un degré d'alcoxylation allant de 20 à 50 OE, de préférence de 20 à 45.

7. Système selon l'une quelconque des revendications précédentes, dans lequel Agent comprend un autre agent tensioactif non ionique.

8. Système selon la revendication précédente, dans lequel l'autre agent tensioactif non ionique est un alcoxylate d'alcool gras, de préférence avec un degré d'alcoxylation de 5 à 15 EO.

9. Système selon la revendication précédente, dans lequel l'alcoxylate d'alcool gras comprend un groupe alkyle en C9 à C12, et dans lequel le groupe alkyle est de préférence un groupe alkyle non ramifié.

10. Fabrication d'un système selon la revendication précédente, comprenant les étapes consistant à,
Rendre le gel plus fluide en le chauffant,
Introduction du produit dans le panier,
Refroidissement de la composition, ce qui permet à l'agent de durcir.
